# EUROPÄISCHE PATENTSCHRIFT

(11) **EP 3 649 936 B1**
(45) Veröffentlichungstag und Bekanntmachung des Hinweises auf die Patenterteilung: **29.03.2023**
(21) Anmeldenummer: 19000509.0
(22) Anmeldetag: 06.11.2019
(51) Int. Cl.: A61B 5/097, A61M 16/08, A61M 16/00, A61M 16/10

(54) **WASSERFALLE FÜR EIN BEATMUNGSGERÄT UND BEATMUNGSGERÄT MIT WASSERFALLE**
WATER TRAP FOR A VENTILATOR AND VENTILATOR WITH WATER TRAP
CASCADE POUR UN APPAREIL RESPIRATOIRE ET APPAREIL RESPIRATOIRE POURVU DE CASCADE

(30) Priorität: 07.11.2018 DE 102018008729
(43) Veröffentlichungstag der Anmeldung: 13.05.2020
(73) Patentinhaber: Löwenstein Medical Technology S.A., 2557 Luxembourg (LU)
(72) Erfinder: Petrus, Johannes Josephus Borm, 5627 Eindhoven (NL)
(74) Vertreter: Marx, Thomas

(56) Entgegenhaltungen:
- EP-A1- 2 457 506
- WO-A1-2012/094008
- WO-A1-2014/027290
- DE-A1-102012 220 565
- DE-C1- 19 901 590
- US-A1- 2008 119 754
- US-B2- 7 940 175

## Beschreibung

Diese Erfindung bezieht sich allgemein auf das Gebiet der Beatmungsgeräte, die Atemgas konditionieren.

Darunter ist beispielsweise eine Anfeuchtung von Atemgas zu verstehen oder auch eine Zumischung von Gasen wie Sauerstoff oder von Narkosegasen. Solche Beatmungsgeräte können in klinischen Anwendungen als Narkose-Beatmungsgerät ausgeführt sein oder in einem heimischen Umfeld als Beatmungsgerät mit Anfeuchtung und/oder Sauerstoffzufuhr.

Sogenannte Wasserfallen dienen dazu, überschüssige Feuchtigkeit zu entfernen. Beispielsweise werden solche Wasserfallen einem Gasanalysator, der die Anteile der Atemgase oder Narkosegase bestimmen soll, vorgeschaltet.

Die Einstellung und Einhaltung definierter Vorgabewerte für Atemgase oder Narkosegase oder Feuchtigkeit ist dabei oftmals problematisch.

Im Stand der Technik sind verschiedene Systeme zur Atemgasanalyse mit vorgeschalteten Wasserfallen bekannt.

Die DE 199 01 590 C1 offenbart eine Wasserfalle, die über eine Kanüle entleert werden kann. Die WO 2012/094008 A1 zeigt neben einem System zur Förderung von Atemgas auch eine Ausführung zur Analyse von Atemgas mit einem Abgriff von der Atemgasleitung sowie einer Wasserfalle zwischen dem Abgriff und dem Gasanalysator.

Die EP 2 457 506 A1 sieht eine lösbare Wasserfalle zwischen Abgriff und Gasanalysator vor, wobei die Filter der Wasserfalle mehrere verschiedene Beschichtungen aufweisen um verschiedene Bestandteile des Atemgases herauszufiltern.

Eine Gasprobenleitung zur Vortrocknung des Atemgases vor dem Gasanalysator wird von der DE 10 2012 2220 565 A1 vorgeschlagen. Zusätzlich kann hier eine Wasserfalle zwischengeschaltet sein.

Die US 7,940,175 B2 beschreibt eine Wasserfalle, welche von dem System zur Gasanalyse erkannt werden kann und zum Teil auch den Füllstand der Wasserfalle ermitteln kann.

Aus der WO 2014/027290 A1 ist eine wechselbare Wasserfalle bekannt, welche in einer Reihe verschiedener Leitungen zwischen geschaltet ist und entsprechend geformte Anschlüsse aufweist.

Daher wäre ein verbessertes (Narkose-)Beatmungsgerät von Vorteil und insbesondere wäre eine erhöhte Betriebssicherheit und / oder Genauigkeit von Vorteil. Deshalb versuchen Ausführungsformen der vorliegenden Erfindung vorzugsweise, einen oder mehrere Mängel, Nachteile oder Probleme des Standes der Technik, wie beispielsweise die oben genannten, einzeln oder in einer beliebigen Kombination zu verbessern, zu mildern oder zu beseitigen, indem ein Beatmungsgerät, ein Verfahren und eine Vorrichtung bereitgestellt werden gemäß den beigefügten Patentansprüchen.

Die Erfindung betrifft ein Beatmungsgerät mit einem Gasanalysator und einem Schlauchsystem für eine gasleitende Verbindung von dem Beatmungsgerät zu einem Patienten und zu dem Gasanalysator, wobei das Schlauchsystem einen Abgriff aufweist an den eine Wasserfalle lösbar konnektiert ist, wobei die Wasserfalle ein Anschlussmittel für die Verbindung mit dem Abgriff aufweist, wobei das Anschlussmittel in einen Schlauch übergeht, der eine gasleitende Verbindung von dem Schlauchsystem in die Wasserfalle ermöglicht, wobei der Schlauch mit der Wasserfalle verbunden ist und wobei die Wasserfalle einen vorderen Teilbereich aufweist und einen hinteren Teilbereich aufweist, der dem Beatmungsgerät zugewandt ist, wobei das Beatmungsgerät eine Aufnahme für die Wasserfalle aufweist, zur lösbaren Verbindung mit der Wasserfalle , wobei die Wasserfalle gasleitend mit dem Beatmungsgerät verbunden ist und wobei der Gasanalysator gasleitend mit der Aufnahme und der Wasserfalle verbunden ist, wobei der Gasanalysator eine Saugeinheit aufweist, die eingerichtet ist, einen definierten Gasstrom aus dem Schlauchsystem über die Wasserfalle zu dem Gasanalysator zu fördern, dadurch gekennzeichnet, dass die Wasserfalle einen unteren Teilbereich aufweist, der ein Wasserreservoir bildet, wobei der untere Teilbereich eine Entnahmestelle aufweist, für die Entnahme von Wasser aus dem Reservoir mittels einer Kanüle.

Die Erfindung ist auch dadurch gekennzeichnet, dass die Saugeinheit einen vorgebbaren Unterdruck erzeugt und damit einen Gasstrom aus dem Schlauchsystem über die Wasserfalle zu dem Gasanalysator fördert, wobei die Saugeinheit einen Drucksensor und/oder Flusssensor aufweist zur Bestimmung des Unterdruckes und/oder eines Gasflusses.

Die Erfindung ist auch dadurch gekennzeichnet, dass der Drucksensor, mit der die Steuereinheit verbunden ist, die, bei einer definierten Abweichung des Unterdruckes von einem Vorgabewert, einen Alarm auslöst.

Die Erfindung ist auch dadurch gekennzeichnet, dass der Flusssensor, mit dem die Steuereinheit verbunden ist, die, bei einer definierten Abweichung des Gasflusses von einem Vorgabewert, einen Alarm auslöst.

Die Erfindung ist auch dadurch gekennzeichnet, dass das Beatmungsgerät zumindest einen Lese-/Schreibgerät für Speicherbausteine aufweist.

Die Erfindung ist auch dadurch gekennzeichnet, dass die Wasserfalle eingerichtet und ausgebildet ist, lösbar mit einer Aufnahme des Beatmungsgerätes und lösbar mit dem Schlauchsystem verbunden zu werden.

Die Erfindung betrifft auch eine Wasserfalle zur Verwendung mit einem Beatmungsgerät, wobei die Wasserfalle eingerichtet und ausgebildet ist lösbar mit einer Aufnahme des Beatmungsgerätes und lösbar mit dem Schlauchsystem verbunden zu werden, dadurch gekennzeichnet, dass die Wasserfalle einen unteren Teilbereich aufweist, der ein Wasserreservoir bildet, wobei der untere Teilbereich eine Entnahmestelle aufweist, für die Entnahme von Wasser aus dem Reservoir mittels einer Kanüle.

Die Erfindung ist auch dadurch gekennzeichnet, dass der Schlauch zumindest abschnittsweise von einem Schutzschlauch umgeben ist, wobei der Schutzschlauch zumindest abschnittsweise koaxial zu dem Schlauch verläuft.

Die Erfindung ist auch dadurch gekennzeichnet, dass der Schutzschlauch unlösbar mit der Wasserfalle verbunden ist.

Die Erfindung ist auch dadurch gekennzeichnet, dass der Schutzschlauch formschlüssig oder stoffschlüssig mit der Wasserfalle verbunden ist.

Die Erfindung ist auch dadurch gekennzeichnet, dass die Wasserfalle einen unteren Teilbereich aufweist, der das Wasserreservoir bildet.

Die Erfindung ist auch dadurch gekennzeichnet, dass der untere Teilbereich eine Entnahmestelle aufweist, für die Entnahme von Wasser aus dem Reservoir mittels einer Kanüle.

Die Erfindung ist auch dadurch gekennzeichnet, dass die Wasserfalle, benachbart zu der Einmündung des Schlauches in die Wasserfalle, zumindest ein Filterelement aufweist, welches so eingerichtet und ausgebildet ist, dass eine Gasströmung durch die Wasserfalle in den Gasanalysator zumindest in einem Funktionszustand ausschließlich durch das Filterelement erfolgt.

Die Erfindung ist auch dadurch gekennzeichnet, dass das Filterelement zwischen dem vorderen Teilbereich und dem hinteren Teilbereich der Wasserfalle angeordnet ist.

Die Erfindung ist auch dadurch gekennzeichnet, dass der hintere Teilbereich der Wasserfalle zumindest eine Aufnahme für das Filterelement aufweist.

Die Erfindung ist auch dadurch gekennzeichnet, dass der hintere Teilbereich der Wasserfalle im Bereich der Aufnahme Strömungsleitelemente für das Filterelement aufweist.

Die Erfindung ist auch dadurch gekennzeichnet, dass der hintere Teilbereich der Wasserfalle zumindest einen Stutzen aufweist, der komplementär zu einer Öffnung in der Aufnahme ausgeführt ist.

Die Erfindung ist auch dadurch gekennzeichnet, dass der hintere Teilbereich der Wasserfalle zumindest einen Stutzen aufweist, der komplementär zu einer Öffnung in der Aufnahme ausgeführt ist.

Die Erfindung ist auch dadurch gekennzeichnet, dass die Wasserfalle, benachbart zu der Einmündung des Schlauches in die Wasserfalle, zumindest ein Filterelement und benachbart dazu ein zweites Filterelement aufweist, wobei die Filterelemente, beispielsweise über einen Bypass, derart gasleitend miteinander verbunden sind, dass bei Zunahme des Strömungswiderstandes des ersten Filterelementes , über einen Schwellenwert hinaus, eine gasleitende Verbindung von dem Schlauch über den Bypass in das zweite Filterelement und zu dem Gasanalysator erfolgt.

Die Erfindung ist auch dadurch gekennzeichnet, dass die Wasserfalle einen Speicherbaustein aufweist, der zumindest Nutzungsdaten oder Herstellungsdaten der Wasserfalle speichert.

Die Erfindung ist auch dadurch gekennzeichnet, dass die Wasserfalle beidseitige Federlaschen aufweist die komplementär zu Rastmitteln im Bereich der Aufnahme ausgebildet sind.

Die Erfindung ist auch dadurch gekennzeichnet, dass die Federlaschen Bedienflächen und einen Kragen aufweisen.

Die Erfindung ist auch dadurch gekennzeichnet, dass die Rastmittel Sicherungselemente aufweisen, die in einem Funktionszustand zumindest teilweise den Kragen überlappen und somit sichern.

Die Erfindung ist auch dadurch gekennzeichnet, dass die Wasserfalle benachbart zu dem Federlaschen Aussparungen aufweist die komplementär zu den Federlaschen ausgebildet sind, wobei die Aussparungen den Federraum für die Federlaschen bilden.

Die Erfindung ist auch dadurch gekennzeichnet, dass die Federlaschen und die Rastmittel eine Klick-Geräusch generierende Gestaltung aufweisen, die ein Einrasten der Federlaschen im Bereich der Rastmittel akustisch wahrnehmbar machen.

Die Erfindung betrifft auch ein Verfahren zur Analyse von Beatmungsgas umfassend ein Beatmungsgerät mit einem Gasanalysator und einem Schlauchsystem, für eine gasleitende Verbindung von dem Beatmungsgerät zu einem Patienten und zu dem Gasanalysator, wobei das Schlauchsystem einen Abgriff aufweist an den eine Wasserfalle lösbar konnektiert ist, wobei die Wasserfalle ein Anschlussmittel für die Verbindung mit dem Abgriff aufweist, wobei das Anschlussmittel in einen Schlauch übergeht, der eine gasleitende Verbindung von dem Schlauchsystem in die Wasserfalle ermöglicht, wobei der Schlauch mit der Wasserfalle verbunden ist und wobei die Wasserfalle einen vorderen Teilbereich ausweist und einen hinteren Teilbereich aufweist, der dem Beatmungsgerät zugewandt ist, wobei das Beatmungsgerät eine Aufnahme für die Wasserfalle aufweist, zur lösbaren Verbindung mit der Wasserfalle, wobei die Wasserfalle gasleitend mit dem Beatmungsgerät verbunden ist und wobei der Gasanalysator gasleitend mit der Aufnahme und der Wasserfalle verbunden ist, wobei der Gasanalysator eine Saugeinheit aufweist, die eingerichtet ist, einen definierten Gasstrom aus dem Schlauchsystem über die Wasserfalle zu dem Gasanalysator zu fördern dadurch gekennzeichnet, dass die Wasserfalle einen unteren Teilbereich aufweist, der ein Wasserreservoir bildet, wobei der untere Teilbereich eine Entnahmestelle aufweist, für die Entnahme von Wasser aus dem Reservoir mittels einer Kanüle.

Die Erfindung ist auch dadurch gekennzeichnet, dass die Saugeinheit einen vorgebbaren Unterdruck erzeugt und damit einen Gasstrom aus dem Schlauchsystem über die Wasserfalle zu dem Gasanalysator fördert, wobei die Saugeinheit einen Drucksensor und/oder Flusssensor aufweist zur Bestimmung des Unterdruckes und/oder eines Gasflusses.

Die Erfindung ist auch dadurch gekennzeichnet, dass der Drucksensor, mit der die Steuereinheit verbunden ist, die, bei einer definierten Abweichung des Unterdruckes von einem Vorgabewert, einen Alarm auslöst.

Die Erfindung ist auch dadurch gekennzeichnet, dass der Flusssensor, mit dem die Steuereinheit verbunden ist, die, bei einer definierten Abweichung des Gasflusses von einem Vorgabewert, einen Alarm auslöst.

Die Erfindung ist auch dadurch gekennzeichnet, dass das Beatmungsgerät zumindest einen Lese-/Schreibgerät für Speicherbausteine aufweist, wobei zumindest ein Speicherbaustein in der Wasserfalle angeordnet ist und wobei das Lese-/Schreibgerät den Speicherbaustein der Wasserfalle dann erkennt, wenn die Wasserfalle gasleitend mit der Aufnahme und der Wasserfalle verbunden ist.

Die Erfindung ist auch dadurch gekennzeichnet, dass das Beatmungsgerät zumindest einen Lese-/Schreibgerät für Speicherbausteine aufweist, wobei zumindest ein Speicherbaustein in der Wasserfalle angeordnet ist und wobei das Lese-/Schreibgerät den Speicherbaustein der Wasserfalle dann erkennt, wenn die Wasserfalle gasleitend mit der Aufnahme verbunden ist.

Die Erfindung ist auch dadurch gekennzeichnet, dass das Lese-/Schreibgerät eingerichtet und ausgebildet ist den Speicherbaustein der Wasserfalle zu erkennen und bei Erkennung eine Änderung der Funktionsweise von Saugeinheit und/oder Gasanalysator und/oder Gerät veranlasst.

Die Erfindung ist auch dadurch gekennzeichnet, dass das Lese-/Schreibgerät bei Erkennung des Speicherbausteins der Wasserfalle die Saugeinheit aktiviert einen definierten Gasstrom aus dem Schlauchsystem über die Wasserfalle zu dem Gasanalysator zu fördern.

Die Erfindung ist auch dadurch gekennzeichnet, dass das Lese-/Schreibgerät bei Nicht-Erkennung des Speicherbausteins der Wasserfalle die Saugeinheit deaktiviert.

Die Erfindung ist auch dadurch gekennzeichnet, dass zur Ermittlung ob die Wasserfalle gasleitend mit der Aufnahme verbunden ist die Saugeinheit einen vorgebbaren Unterdruck erzeugt und sensorisch den resultierenden Unterdruck und/oder den Gasfluss bestimmt und bei einer definierten Abweichung des Unterdruckes oder des Gasflusses von einem Vorgabewert zusätzlich eine Erkennung des Speicherbausteins der Wasserfalle durch das Lese-/Schreibgerät durchgeführt wird.

Die Erfindung ist auch dadurch gekennzeichnet, dass zur Ermittlung ob die Wasserfalle gasleitend mit der Aufnahme verbunden ist eine Erkennung des Speicherbausteins der Wasserfalle durch das Lese-/Schreibgerät durchgeführt wird und dann die Saugeinheit einen vorgebbaren Unterdruck erzeugt und sensorisch den resultierenden Unterdruck und/oder den Gasfluss bestimmt.

Gemäß einem Aspekt der Erfindung wird ein Beatmungsgerät bereitgestellt mit einem Gasanalysator und einem Schlauchsystem, für eine gasleitende Verbindung von dem Beatmungsgerät zu einem Patienten und zu dem Gasanalysator wobei das Schlauchsystem einen Abgriff aufweist an den eine Wasserfalle lösbar konnektiert ist, wobei die Wasserfalle ein Anschlussmittel für die Verbindung mit dem Abgriff aufweist, wobei das Anschlussmittel in einen Schlauch übergeht, der eine gasleitende Verbindung von dem Schlauchsystem in die Wasserfalle ermöglicht, wobei der Schlauch mit der Wasserfalle verbunden ist und wobei die Wasserfalle einen vorderen Teilbereich ausweist und einen hinteren Teilbereich aufweist, der dem Beatmungsgerät zugewandt ist, wobei das Beatmungsgerät eine Aufnahme für die Wasserfalle aufweist, zur lösbaren Verbindung mit der Wasserfalle, wobei die Wasserfalle gasleitend mit dem Beatmungsgerät verbunden ist und wobei der Gasanalysator gasleitend mit der Aufnahme und der Wasserfalle verbunden ist, wobei der Gasanalysator eine Saugeinheit aufweist, die eingerichtet ist, einen definierten Gasstrom aus dem Schlauchsystem über die Wasserfalle zu dem Gasanalysator zu fördern.

Gemäß noch einem weiteren Aspekt der Erfindung wird eine Wasserfalle zur Verwendung mit einem Beatmungsgerät bereitgestellt. Wobei die Wasserfalle eingerichtet und ausgebildet ist, lösbar mit einer Aufnahme des Beatmungsgerätes und lösbar mit dem Schlauchsystem verbunden zu werden.

Gemäß noch einem weiteren Aspekt der Erfindung wird ein Verfahren bereitgestellt. Das Verfahren dient zur Analyse von Beatmungsgas und umfasst ein Beatmungsgerät mit einem Gasanalysator und einem Schlauchsystem, für eine gasleitende Verbindung von dem Beatmungsgerät zu einem Patienten und zu dem Gasanalysator, wobei das Schlauchsystem einen Abgriff für eine Wasserfalle aufweist, wobei die Wasserfalle ein Anschlussmittel für die Verbindung mit dem Abgriff aufweist, wobei das Anschlussmittel in einen Schlauch übergeht, der eine gasleitende Verbindung von dem Schlauchsystem in die Wasserfalle ermöglicht, wobei der Schlauch mit der Wasserfalle verbunden ist und wobei die Wasserfalle einen vorderen Teilbereich ausweist und einen hinteren Teilbereich aufweist, der dem Beatmungsgerät zugewandt ist, wobei das Beatmungsgerät eine Aufnahme für die Wasserfalle aufweist, zur lösbaren Verbindung mit der Wasserfalle, wobei die Wasserfalle gasleitend mit dem Beatmungsgerät verbunden ist und wobei der Gasanalysator gasleitend mit der Aufnahme und der Wasserfalle verbunden ist, wobei der Gasanalysator eine Saugeinheit aufweist, die eingerichtet ist, einen definierten Gasstrom aus dem Schlauchsystem über die Wasserfalle zu dem Gasanalysator zu fördern.

Gemäß noch einem weiteren Aspekt der Erfindung wird ein weiteres Verfahren bereitgestellt. Dieses Verfahren ist dadurch gekennzeichnet, dass ein Lese-/Schreibgerät des Beatmungsgerätes eingerichtet und ausgebildet ist den Speicherbaustein der Wasserfalle zu erkennen und bei Erkennung eine Änderung der Funktionsweise von Saugeinheit und/oder Gasanalysator und/oder Beatmungsgerät zu veranlassen.

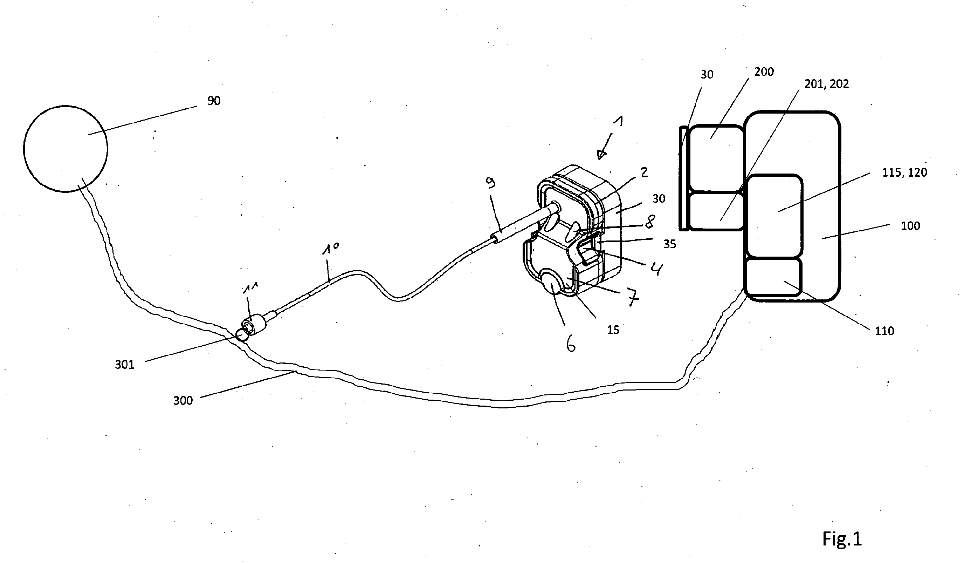

Figur 1 zeigt die Wasserfalle 1 mit einem Schlauch 10 und einem Anschlussmittel 11 für die Verbindung mit dem Schlauchsystem, womit eine gasleitende Verbindung von dem Schlauchsystem 300 in die Wasserfalle 1 ermöglicht wird. Die Wasserfalle 1 wird von einer Aufnahme 30 lösbar gehalten. Die Aufnahme 30 kann Teil eines Beatmungsgerätes sein oder an das Beatmungsgerät angefügt werden. Das Beatmungsgerät 100 kann als klinisches Beatmungsgerät oder als Anästhesie-Beatmungsgerät ausgeführt sein. Das Beatmungsgerät kann alternativ auch als Heimbeatmungsgerät ausgeführt sein.

Das Beatmungsgerät 100 weist einen Gasanalysator 200 und ein Schlauchsystem 300 auf, für eine gasleitende Verbindung von dem Beatmungsgerät 100 zu einem Patienten 90 und zu dem Gasanalysator 200. Das Schlauchsystem 300 weist einen Abgriff 301 auf an den eine Wasserfalle 1 lösbar konnektiert ist, wobei die Wasserfalle 1 ein Anschlussmittel 11 für die Verbindung mit dem Abgriff 301 aufweist. Das Anschlussmittel 11 geht in einen Schlauch 10 über, der eine gasleitende Verbindung von dem Schlauchsystem 300 in die Wasserfalle 1 ermöglicht. Das Beatmungsgerät weist eine Aufnahme 30 für die Wasserfalle 1 auf, zur kraftschlüssigen und/oder formschlüssigen lösbaren Verbindung mit der Wasserfalle 1, wobei die Wasserfalle 1 gasleitend mit dem Beatmungsgerät verbunden ist und wobei der Gasanalysator gasleitend mit der Aufnahme 30 und der Wasserfalle 1 verbunden ist, wobei der Gasanalysator 200 eine Saugeinheit 201 aufweist, die eingerichtet ist, einen definierten Gasstrom aus dem Schlauchsystem 300 über die Wasserfalle 1 zu dem Gasanalysator 200 zu fördern.

Die Saugeinheit erzeugt einen vorgebbaren Unterdruck und fördert damit einen Gasstrom aus dem Schlauchsystem 300 über die Wasserfalle 1 zu dem Gasanalysator 200, wobei die Saugeinheit einen Drucksensor und/oder Flusssensor 202 aufweist zur Bestimmung des Unterdruckes und/oder eines Gasflusses.

Der Drucksensor 202 und/oder Flusssensor ist mit der Steuereinheit 120 verbunden die, bei einer definierten Abweichung des Unterdruckes/Gasflusses von einem Vorgabewert, einen Alarm auslöst.

Das Beatmungsgerät weist zumindest ein Lese-/Schreibgerät 115 für Speicherbausteine 15 auf. Die Wasserfalle weist einen Speicherbaustein 15 auf, der zumindest Nutzungsdaten oder Herstellungsdaten der Wasserfalle speichert.

Das Lese-/Schreibgerät 115 erkennt den Speicherbaustein der Wasserfalle dann, wenn die Wasserfalle gasleitend mit der Aufnahme 30 und der Wasserfalle 1 verbunden ist.

Bei Erkennung des Speicherbausteins 15 der Wasserfalle aktiviert das Lese-/Schreibgerät 115 die Saugeinheit 201 einen definierten Gasstrom aus dem Schlauchsystem 300 über die Wasserfalle 1 zu dem Gasanalysator 200.

Das Lese-/Schreibgerät 115 deaktiviert bei Nicht-Erkennung des Speicherbausteins 15 der Wasserfalle die Saugeinheit 201.

Zur Ermittlung ob die Wasserfalle gasleitend mit der Aufnahme 30 verbunden ist, erzeugt die Saugeinheit einen vorgebbaren Unterdruck und bestimmt sensorisch den resultierenden Unterdruck und/oder den Gasfluss. Bei einer definierten Abweichung des Unterdruckes oder des Gasflusses von einem Vorgabewert wird zusätzlich eine Erkennung des Speicherbausteins 15 der Wasserfalle durch das Lese-/Schreibgerät 115 durchgeführt.

Zur Ermittlung ob die Wasserfalle gasleitend mit der Aufnahme 30 verbunden ist erfolgt alternativ oder ergänzend eine Erkennung des Speicherbausteins 15 der Wasserfalle durch das Lese-/Schreibgerät 115. Daraufhin erzeugt die Saugeinheit einen Unterdruck und bestimmt sensorisch den resultierenden Unterdruck und/oder den Gasfluss.

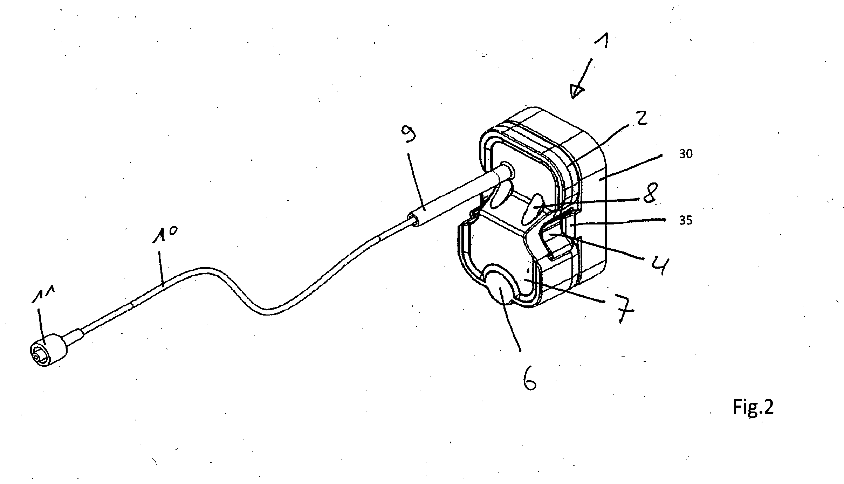

Figur 2 zeigt die Wasserfalle 1 mit einem Schlauch 10 und einem Anschlussmittel 11 für die Verbindung mit dem Schlauchsystem, womit eine gasleitende Verbindung von dem Schlauchsystem 300 in die Wasserfalle 1 ermöglicht wird. Die Wasserfalle 1 wird von einer Aufnahme 30 lösbar gehalten. Das Anschlussmittel 11 ist bevorzugt unlösbar mit dem Schlauch 10 verbunden ist und der Schlauch 10 ist unlösbar mit der Wasserfalle verbunden.

Der Schlauch 10 ist abschnittsweise von einem Schutzschlauch 9 umgeben, wobei der Schutzschlauch zumindest abschnittsweise koaxial zu dem Schlauch 10 verläuft. Der Schlauch und/oder der Schutzschlauch 9 sind formschlüssig oder stoffschlüssig mit der Wasserfalle 1 verbunden.

Die Wasserfalle weist einen unteren Teilbereich 7 auf, der das Wasserreservoir bildet. Ein Wandbereich der Wasserfalle 1, benachbart zu dem Wasserreservoir 7, ist als eine Entnahmestelle 6 ausgebildet. Eine Entnahme von Wasser, für Probenzwecke oder für die Entleerung, ist mittels einer Kanüle möglich, die durch die Entnahmestelle eingestochen wird.

Die Wasserfalle 1 weist beidseitige Federlaschen 4 auf, die komplementär zu Rastmitteln 35 im Bereich der Aufnahme 30 ausgebildet sind. Benachbart zu dem Federlaschen 4 weist die Wasserfalle Aussparungen 3 auf, die komplementär zu den Federlaschen 4 ausgebildet sind, wobei die Aussparungen 3 den Federraum 5 für die Federlaschen 4 bilden.

Die Federlaschen 4 und die Rastmitteln 35 weisen eine Klick-Geräusch generierende Gestaltung auf, die ein Einrasten der Federlaschen 4 im Bereich der Rastmittel 35 akustisch wahrnehmbar machen.

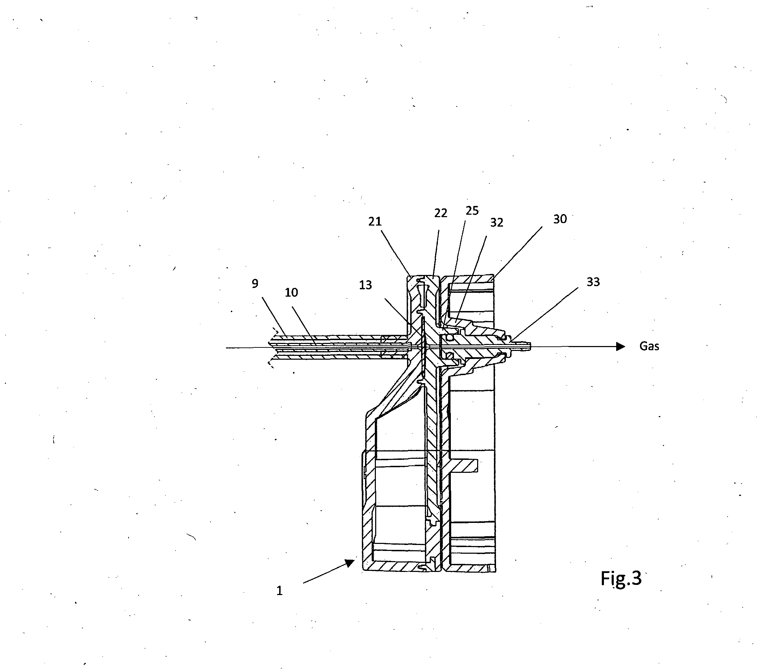

Figur 3 zeigt die Wasserfalle 1 mit dem Schlauch 10 und die gasleitende Verbindung durch die Wasserfalle 1. Benachbart zu der Einmündung des Schlauches 10 in die Wasserfalle ist zumindest ein Filterelement 13 angeordnet, welches so eingerichtet und ausgebildet ist, dass eine Gasströmung durch die Wasserfalle in den Gasanalysator zumindest in einem Funktionszustand ausschließlich durch das Filterelement 13 erfolgt.

Das Filterelement 13 ist zwischen dem vorderen Teilbereich 21 und dem hinteren Teilbereich 22 der Wasserfalle angeordnet.

Der hintere Teilbereich 22 der Wasserfalle weist zumindest einen Stutzen 25 auf, der komplementär zu einer Öffnung 32 in der Aufnahme 30 ausgeführt ist. Ein Ventil 33 ermöglicht den Durchfluss von Gas durch die Aufnahme in Richtung des Gasanalysators.

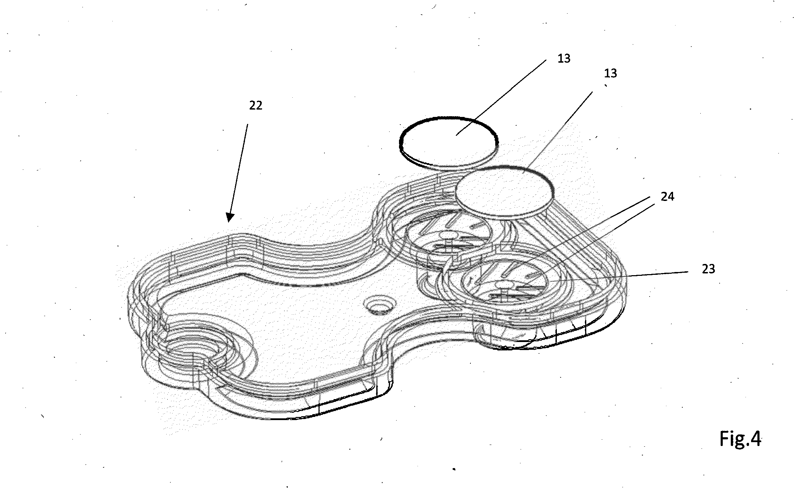

Figur 4 zeigt die Wasserfalle 1 mit den Filterelementen. Der hintere Teilbereich 22 der Wasserfalle weist zumindest eine Aufnahme 23 für Filterelemente 13, 13' auf. Im Bereich der Aufnahme 23 sind Strömungsleitelemente 24 für das Filterelement 13 vorgesehen.

Der hintere Teilbereich 22 der Wasserfalle weist zumindest einen Stutzen 25 auf, der komplementär zu einer Öffnung 32 in der Aufnahme 30 ausgeführt ist.

Aus den Figuren 2 und 4 wird deutlich: Benachbart zu der Einmündung des Schlauches 10 in die Wasserfalle sind zwei Filterelemente 13, 13' vorgesehen, wobei die Filterelemente über einen Bypass 8 derart gasleitend miteinander verbunden sind, dass bei Zunahme des Strömungswiderstandes des ersten Filterelementes 13, über einen Schwellenwert hinaus, eine gasleitende Verbindung von dem Schlauch über den Bypass in das zweite Filterelement 13' und zu dem Gasanalysator erfolgt.

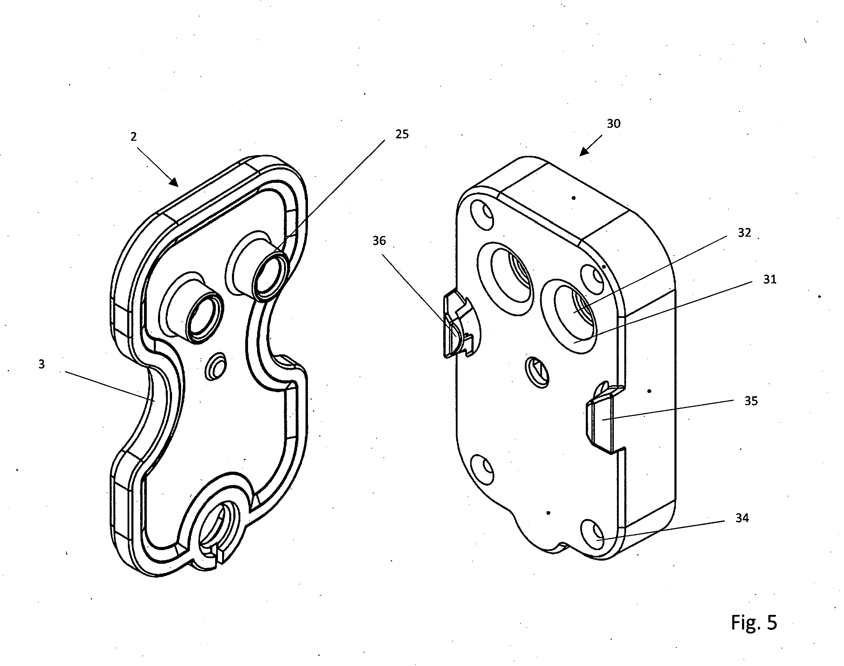

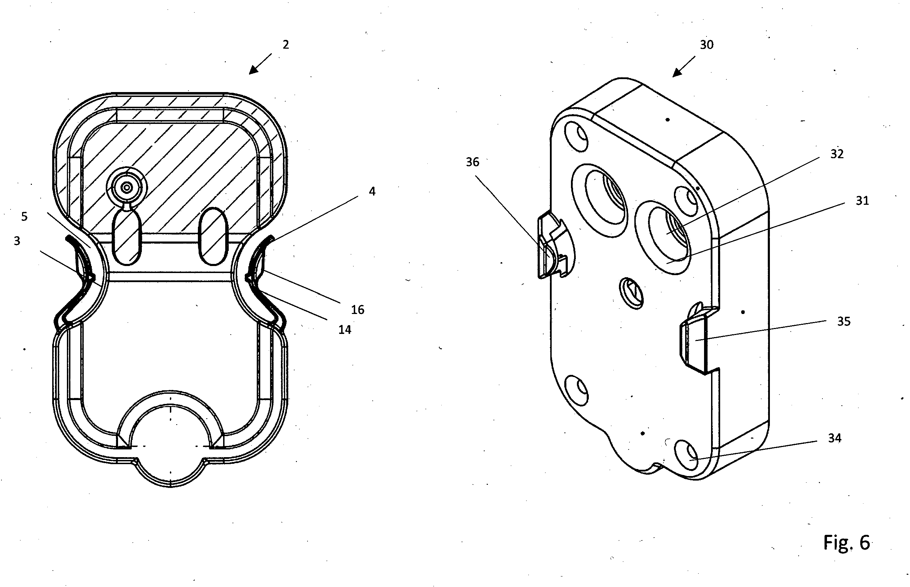

Aus den Figuren 5 und 6 wird deutlich: Die Aufnahme 30 weist Befestigungsmittel 34 auf zur Befestigung der Aufnahme an dem Beatmungsgerät. Die Aufnahme weist zudem Rastmittel 35, 36 auf zur Halterung der Wasserfalle. Die Öffnungen 32 in der Aufnahme dienen der zumindest teilweisen Aufnahme von Stutzen 25 der Wasserfalle 2. Die Öffnungen weisen beispielsweise Einführschrägen 31 für die Stützen auf.

Die Federlaschen 4 der Wasserfalle befinden sich im Bereich einer Einbuchtung 3. Die Federlaschen weisen Bedienflächen 14 und einen Kragen 16 auf.

Die Rastmittel 35 weisen Sicherungselemente 36 auf, die in einem Funktionszustand zumindest teilweise den Kragen 16 der Wasserfalle überlappen und somit die Wasserfalle in der Aufnahme 30 sichern.

## Patentansprüche

1. Beatmungsgerät (100) mit einem Gasanalysator (200) und einem Schlauchsystem (300), für eine gasleitende Verbindung von dem Beatmungsgerät (100) zu einem Patienten (90) und zu dem Gasanalysator (200), wobei das Schlauchsystem (300) einen Abgriff (301) aufweist an den eine Wasserfalle (1) lösbar konnektiert ist, wobei die Wasserfalle (1) ein Anschlussmittel (11) für die Verbindung mit dem Abgriff (301) aufweist, wobei das Anschlussmittel (11) in einen Schlauch (10) übergeht, der eine gasleitende Verbindung von dem Schlauchsystem (300) in die Wasserfalle (1) ermöglicht, wobei der Schlauch mit der Wasserfalle (1) verbunden ist und wobei die Wasserfalle einen vorderen Teilbereich aufweist (21) und einen hinteren Teilbereich (22) aufweist, der dem Beatmungsgerät (100) zugewandt ist, wobei das Beatmungsgerät eine Aufnahme (30) für die Wasserfalle (1) aufweist, zur lösbaren Verbindung mit der Wasserfalle (1), wobei die Wasserfalle (1) gasleitend mit dem Beatmungsgerät verbunden ist und wobei der Gasanalysator gasleitend mit der Aufnahme (30) und der Wasserfalle (1) verbunden ist, wobei der Gasanalysator (200) eine Saugeinheit (201) aufweist, die eingerichtet ist, einen definierten Gasstrom aus dem Schlauchsystem (300) über die Wasserfalle (1) zu dem Gasanalysator (200) zu fördern, **dadurch gekennzeichnet, dass** die Wasserfalle einen unteren Teilbereich (7) aufweist, der ein Wasserreservoir bildet, wobei der untere Teilbereich (7) eine Entnahmestelle (6) aufweist, für die Entnahme von Wasser aus dem Reservoir mittels einer Kanüle.

2. Beatmungsgerät nach Anspruch 1 **dadurch gekennzeichnet, dass** die Saugeinheit einen vorgebbaren Unterdruck erzeugt und damit einen Gasstrom aus dem Schlauchsystem (300) über die Wasserfalle (1) zu dem Gasanalysator (200) fördert, wobei die Saugeinheit einen Drucksensor und/oder Flusssensor (202) aufweist zur Bestimmung des Unterdruckes und/oder eines Gasflusses.

3. Beatmungsgerät nach Anspruch 2 **dadurch gekennzeichnet, dass** der Drucksensor (202) und/oder der Flusssensor, mit der Steuereinheit (120) verbunden sind, wobei die Steuereinheit (120), bei einer definierten Abweichung des Unterdruckes von einem Vorgabewert, einen Alarm auslöst.

4. Beatmungsgerät nach zumindest einem der vorhergehenden Ansprüche **dadurch gekennzeichnet, dass** das Beatmungsgerät zumindest einen Lese-/Schreibgerät (115) für Speicherbausteine (15) aufweist.

5. Wasserfalle (1) zur Verwendung mit einem Beatmungsgerät nach Anspruch 1 wobei die Wasserfalle eingerichtet und ausgebildet ist lösbar mit einer Aufnahme (30) des Beatmungsgerätes und lösbar mit dem Schlauchsystem verbunden zu werden, **dadurch gekennzeichnet, dass** die Wasserfalle einen unteren Teilbereich (7) aufweist, der ein Wasserreservoir bildet, wobei der untere Teilbereich (7) eine Entnahmestelle (6) aufweist, für die Entnahme von Wasser aus dem Reservoir mittels einer Kanüle.

6. Wasserfalle nach Anspruch 5 **dadurch gekennzeichnet, dass** das Anschlussmittel (11) unlösbar mit dem Schlauch (10) verbunden ist und der Schlauch (10) unlösbar mit der Wasserfalle verbunden ist.

7. Wasserfalle nach Anspruch 5 oder 6 **dadurch gekennzeichnet, dass** der Schlauch (10) zumindest abschnittsweise von einem Schutzschlauch (9) umgeben ist, wobei der Schutzschlauch zumindest abschnittsweise koaxial zu dem Schlauch (10) verläuft.

8. Wasserfalle nach zumindest einem der Ansprüche 5 bis 7 **dadurch gekennzeichnet, dass** die Wasserfalle, benachbart zu der Einmündung des Schlauches (10) in die Wasserfalle, zumindest ein Filterelement (13) aufweist, welches so eingerichtet und ausgebildet ist, dass eine Gasströmung durch die Wasserfalle in den Gasanalysator zumindest in einem Funktionszustand ausschließlich durch das Filterelement (13) erfolgt.

9. Wasserfalle nach zumindest einem der Ansprüche 5 bis 8 **dadurch gekennzeichnet, dass** die Wasserfalle, benachbart zu der Einmündung des Schlauches (10) in die Wasserfalle, zumindest ein Filterelement (13) und benachbart dazu ein zweites Filterelement (13') aufweist, wobei die Filterelemente, beispielsweise über einen Bypass (8), derart gasleitend miteinander verbunden sind, dass bei Zunahme des Strömungswiderstandes des ersten Filterelementes (13), über einen Schwellenwert hinaus, eine gasleitende Verbindung von dem Schlauch über den Bypass in das zweite Filterelement (13') und zu dem Gasanalysator erfolgt.

10. Wasserfalle nach zumindest einem der Ansprüche 5 bis 9 **dadurch gekennzeichnet, dass** die Wasserfalle einen Speicherbaustein (15) aufweist, der zumindest Nutzungsdaten oder Herstellungsdaten der Wasserfalle speichert.

11. Wasserfalle nach zumindest einem der Ansprüche 5 bis 10 **dadurch gekennzeichnet, dass** die Wasserfalle beidseitige Federlaschen (4) aufweist die komplementär zu Rastmitteln (35) im Bereich der Aufnahme (30) ausgebildet sind, wobei die Wasserfalle benachbart zu den Federlaschen (4) Aussparungen (3) aufweist, die komplementär zu den Federlaschen (4) ausgebildet sind, wobei die Aussparungen (3) den Federraum (5) für die Federlaschen (4) bilden.

12. Wasserfalle nach zumindest einem der Ansprüche 5 bis 11 **dadurch gekennzeichnet, dass** die Federlaschen (4) Bedienflächen (14) und einen Kragen (16) aufweisen, wobei die Rastmittel (35) Sicherungselemente (36) aufweisen, die in einem Funktionszustand zumindest teilweise den Kragen überlappen und somit sichern.

13. Wasserfalle nach zumindest einem der Ansprüche 5 bis 12 **dadurch gekennzeichnet, dass** die Federlaschen (4) und die Rastmitteln (35) eine Klick-Geräusch generierende Gestaltung aufweisen, die ein Einrasten der Federlaschen (4) im Bereich der Rastmittel (35) akustisch wahrnehmbar machen.

14. Verfahren zur Analyse von Beatmungsgas umfassend ein Beatmungsgerät (100) mit einem Gasanalysator (200) und einem Schlauchsystem (300), für eine gasleitende Verbindung von dem Beatmungsgerät (100) zu einem Patienten (90) und zu dem Gasanalysator (200), wobei das Schlauchsystem (300) einen Abgriff (301) aufweist an den eine Wasserfalle (1) lösbar konnektiert ist, wobei die Wasserfalle (1) ein Anschlussmittel (11) für die Verbindung mit dem Abgriff (301) aufweist, wobei das Anschlussmittel (11) in einen Schlauch (10) übergeht, der eine gasleitende Verbindung von dem Schlauchsystem (300) in die Wasserfalle (1) ermöglicht, wobei der Schlauch mit der Wasserfalle (1) verbunden ist und wobei die Wasserfalle einen vorderen Teilbereich ausweist (21) und einen hinteren Teilbereich (22) aufweist, der dem Beatmungsgerät (100) zugewandt ist, wobei das Beatmungsgerät eine Aufnahme (30) für die Wasserfalle (1) aufweist, zur lösbaren Verbindung mit der Wasserfalle (1), wobei die Wasserfalle (1) gasleitend mit dem Beatmungsgerät verbunden ist und wobei der Gasanalysator gasleitend mit der Aufnahme (30) und der Wasserfalle (1) verbunden ist, wobei der Gasanalysator (200) eine Saugeinheit (201) aufweist, die eingerichtet ist, einen definierten Gasstrom aus dem Schlauchsystem (300) über die Wasserfalle (1) zu dem Gasanalysator (200) zu fördern, **dadurch gekennzeichnet, dass** die Wasserfalle einen unteren Teilbereich (7) aufweist, der ein Wasserreservoir bildet, wobei der untere Teilbereich (7) eine Entnahmestelle (6) aufweist, für die Entnahme von Wasser aus dem Reservoir mittels einer Kanüle.

## Claims

1. A ventilator (1) having a gas analyzer (200) and a tube system (300) for a connection suitable for conducting gas from the ventilator (100) to a patient (90) and to the gas analyzer (200), wherein the tube system (300) has a tap (301) to which a water trap (1) is releasably connected, wherein the water trap (1) has a connection means (11) for the connection with the tap (301), wherein the connection means (11) transitions into a tube (10), which enables a connection suitable for conducting gas from the tube system (300) into the water trap (1), wherein the tube is connected to the water trap (1) and wherein the water trap has a front partial region (21) and a rear partial region (22), which is facing the ventilator (100), wherein the ventilator comprises a receptacle (30) for the water trap (1) for a releasable connection with the water trap (1), wherein the water trap (1) is connected to the ventilator in a manner suitable for conducting gas and wherein the gas analyzer is connected to the receptacle (30) and the water trap (1) in a manner suitable for conducting gas, wherein the gas analyzer (200) comprises a suction unit (201) which is configured to convey a defined stream of gas from the tube system (300) to the gas analyzer (200) via the water trap (1), **characterized in that** the water trap comprises a lower partial region (7) which forms a water reservoir, wherein the lower partial region (7) comprises a removal site (6) for the removal of water from the reservoir by means of a cannula.

2. The ventilator according to claim 1, **characterized in that** the suction unit produces a presettable negative pressure and therefore conveys a stream of gas from the tube system (300) to the gas analyzer (200) via the water trap (1), wherein the suction unit comprises a pressure sensor and/or flow sensor (202) to determine the negative pressure and/or a stream of gas.

3. The ventilator according to claim 2, **characterized in that** the pressure sensor (202) and/or the flow sensor are connected to the control unit (120), wherein the control unit (120) triggers an alarm if there is a defined deviation of the negative pressure from a preset value.

4. The ventilator according to at least one of the preceding claims, **characterized in that** the ventilator comprises at least one reading/writing device (115) for memory components (15).

5. A water trap (1) for use with a ventilator according to claim 1, wherein the water trap is configured and designed to be releasably connected with a receptacle (30) of the ventilator and releasably connected with the tube system, **characterized in that** the water trap comprises a lower partial region (7) which forms a water reservoir, wherein the lower partial region (7) comprises a removal site (6) for the removal of water from the reservoir by means of a cannula.

6. The water trap according to claim 5, **characterized in that** the connection means (11) is unreleasably connected to the tube (10) and the tube (10) is unreleasably connected to the water trap.

7. The water trap according to claim 5 or 6, **characterized in that** the tube (10) is surrounded at least in sections by a protective tube (9), wherein the protective tube extends at least in sections coaxially to the tube (10).

8. The water trap according to at least one of claims 5 to 7, **characterized in that** the water trap, adjacent to the junction of the tube (10) into the water trap, comprises at least one filter element (13), which is configured and designed such that at least in a functional state a gas flow through the water trap into the gas analyzer takes place exclusively through the filter element (13).

9. The water trap according to at least one of claims 5 to 8, **characterized in that** the water trap, which is adjacent to the junction of the tube (10) into the water trap, comprises at least one filter element (13) and a second filter element (13') adjacent thereto, wherein the filter elements are connected to each other in a manner suitable for conducting gas, for example by means of a bypass (8), in such a way that when the flow resistance of the first filter element (13) increases beyond a threshold value, a connection suitable for conducting gas takes place from the tube into the second filter element (13') and towards the gas analyzer by means of the bypass.

10. The water trap according to at least one of claims 5 to 9, **characterized in that** the water trap comprises a memory component (15) which stores at least usage data or production data of the water trap.

11. The water trap according to at least one of claims 5 to 10, **characterized in that** the water trap comprises spring shackles (4) on both sides which are designed complementary to locking means (35) in the region of the receptacle (30), wherein the water trap comprises recesses (3) adjacent to the spring shackles (4), which recesses are designed complementary to the spring shackles (4), wherein the recesses (3) form the spring chamber (5) for the spring shackles (4).

12. The water trap according to at least one of claims 5 to 11, **characterized in that** the spring shackles (4) comprise operating areas (14) and a collar (16), wherein the locking means (35) comprise locking elements (36) which overlap at least partially with the collar in a functional state, thereby locking it.

13. The water trap according to at least one of claims 5 to 12, **characterized in that** the spring shackles (4) and the locking means (35) comprise a design that generates a clicking noise, which make the spring shackles (4) locking into place in the region of the locking means (35) acoustically perceptible.

14. A method for analyzing respiratory gas, comprising a ventilator (100) having a gas analyzer (200) and a tube system (300), for a connection suitable for conducting gas from the ventilator (100) to a patient (90) and to the gas analyzer (200), wherein the tube system (300) comprises a tap (301) to which a water trap (1) is releasably connected, wherein the water trap (1) comprises a connection means (11) for the connection with the tap (301), wherein the connection means (11) transitions into a tube (10), which enables a connection suitable for conducting gas from the tube system (300) into the water trap (1), wherein the tube is connected to the water trap (1) and wherein the water trap comprises a front partial region (21) and a rear partial region (22), which is facing the ventilator (100), wherein the ventilator comprises a receptacle (30) for the water trap (1) for a releasable connection with the water trap (1), wherein the water trap (1) is connected to the ventilator in a manner suitable for conducting gas and wherein the gas analyzer is connected to the receptacle (30) and the water trap (1) in a manner suitable for conducting gas, wherein the gas analyzer (200) comprises a suction unit (201) which is configured to convey a defined stream of gas from the tube system (300) to the gas analyzer (200) via the water trap (1), **characterized in that** the water trap comprises a lower partial region (7) which forms a water reservoir, wherein the lower partial region (7) comprises a removal site (6) for the removal of water from the reservoir by means of a cannula.

## Revendications

1. Appareil respiratoire (100) doté d'un analyseur de gaz (200) et d'un système de tuyaux (300), pour une liaison de guidage de gaz allant de l'appareil respiratoire (100) vers un patient (90) et vers l'analyseur de gaz (200), dans lequel le système de tuyaux (300) présente un robinet (301) auquel un piège à eau (1) est raccordé de façon détachable, dans lequel le piège à eau (1) présente un moyen de raccordement (11) pour la liaison avec le robinet (301), dans lequel le moyen de raccordement (11) se prolonge par un tuyau (10) permettant une liaison de guidage de gaz allant du système de tuyaux (300) vers le piège à eau (1), dans lequel le tuyau est relié au piège à eau (1) et dans lequel le piège à eau présente une région partielle avant (21) et une région partielle arrière (22) tournée vers l'appareil respiratoire (100), dans lequel l'appareil respiratoire présente un logement (30) pour le piège à eau (1) pour une liaison détachable avec le piège à eau (1), dans lequel le piège à eau (1) est relié à l'appareil respiratoire de manière à guider le gaz et dans lequel l'analyseur de gaz est relié au logement (30) et au piège à eau (1) de manière à guider le gaz, dans lequel l'analyseur de gaz (200) présente une unité d'aspiration (201) configurée pour transporter un flux de gaz défini hors du système de tuyaux (300) jusqu'à l'analyseur de gaz (200) en passant par le piège à eau (1), **caractérisé en ce que** le piège à eau présente une région partielle inférieure (7) formant un réservoir à eau, dans lequel la région partielle inférieure (7) présente un point de prélèvement (6) pour le prélèvement d'eau à partir du réservoir au moyen d'une canule.

2. Appareil respiratoire selon la revendication 1, **caractérisé en ce que** l'unité d'aspiration produit une pression négative prédéfinissable et transporte ainsi un flux de gaz hors du système de tuyaux (300) jusqu'à l'analyseur de gaz (200) en passant par le piège à eau (1), dans lequel l'unité d'aspiration présente un capteur de pression et/ou un capteur de débit (202) pour la détermination de la pression négative et/ou d'un débit de gaz.

3. Appareil respiratoire selon la revendication 2, **caractérisé en ce que** le capteur de pression (202) et/ou le capteur de débit sont reliés à l'unité de commande (120), dans lequel l'unité de commande (120) déclenche une alarme lors d'un écart défini de la pression négative par rapport à une valeur de consigne.

4. Appareil respiratoire selon l'une au moins des revendications précédentes, **caractérisé en ce que** l'appareil respiratoire présente au moins un appareil de lecture/écriture (115) pour des modules de mémoire (15).

5. Piège à eau (1) destiné à être utilisé avec un appareil respiratoire selon la revendication 1, dans lequel le piège à eau est configuré et conçu pour être relié de façon détachable au logement (30) de l'appareil respiratoire et de façon détachable au système de tuyaux, **caractérisé en ce que** le piège à eau présente une région partielle inférieure (7) formant un réservoir à eau, dans lequel la région partielle inférieure (7) présente un point de prélèvement (6) pour le prélèvement d'eau à partir du réservoir au moyen d'une canule.

6. Piège à eau selon la revendication 5, **caractérisé en ce que** le moyen de raccordement (11) est relié de façon non détachable au tuyau (10) et le tuyau (10) est relié de façon non détachable au piège à eau.

7. Piège à eau selon la revendication 5 ou 6, **caractérisé en ce que** le tuyau (10) est entouré au moins partiellement d'un tuyau de protection (9), dans lequel le tuyau de protection s'étend au moins partiellement coaxialement au tuyau (10).

8. Piège à eau selon l'une au moins des revendications 5 à 7, **caractérisé en ce qu'**à côté de l'embouchure du tuyau (10) dans le piège à eau, le piège à eau présente au moins un élément de filtre (13) configuré et conçu de telle façon qu'un écoulement de gaz à travers le piège à eau vers l'analyseur de gaz s'effectue exclusivement à travers l'élément de filtre (13) dans au moins un état fonctionnel.

9. Piège à eau selon l'une au moins des revendications 5 à 8, **caractérisé en ce qu'**à côté de l'embouchure du tuyau (10) dans le piège à eau, le piège à eau présente au moins un élément de filtre (13) et un deuxième élément de filtre (13') adjacent à celui-ci, dans lequel les éléments de filtre sont reliés l'un à l'autre de manière à guider le gaz, par exemple par le biais d'une dérivation (8), de telle façon que lors d'une augmentation de la résistance à l'écoulement du premier élément de filtre (13) au-delà d'une valeur seuil, une liaison de guidage de gaz est établie à partir du tuyau jusqu'au deuxième élément de filtre (13') et vers l'analyseur de gaz en passant par la dérivation.

10. Piège à eau selon l'une au moins des revendications 5 à 9, **caractérisé en ce que** le piège à eau présente un module de mémoire (15) stockant au moins des données d'utilisation ou des données de fabrication du piège à eau.

11. Piège à eau selon l'une au moins des revendications 5 à 10, **caractérisé en ce que** le piège à eau présente des pattes élastiques (4) bilatérales, lesquelles sont conçues de manière complémentaire à des moyens d'encliquetage (35) dans la région du logement (30), dans lequel le piège à eau présente des évidements (3) adjacents aux pattes élastiques (4), lesquels sont conçus de manière complémentaire aux pattes élastiques (4), dans lequel les évidements (3) forment la chambre de ressort (5) pour les pattes élastiques (4).

12. Piège à eau selon l'une au moins des revendications 5 à 11, **caractérisé en ce que** les pattes élastiques (4) présentent des surfaces de commande (14) et un col (16), dans lequel les moyens d'encliquetage (35) présentent des éléments de fixation (36) chevauchant au moins partiellement le col et fixant ainsi celui-ci.

13. Piège à eau selon l'une au moins des revendications 5 à 12, **caractérisé en ce que** les pattes élastiques (4) et les moyens d'encliquetage (35) présentent une conception générant un son de clic permettant de constater acoustiquement un encliquetage des pattes élastiques (4) dans la région des moyens d'encliquetage (35).

14. Procédé d'analyse d'un gaz respiratoire comportant un appareil respiratoire (100) doté d'un analyseur de gaz (200) et d'un système de tuyaux (300), pour une liaison de guidage de gaz allant de l'appareil respiratoire (100) vers un patient (90) et vers l'analyseur de gaz (200), dans lequel le système de tuyaux (300) présente un robinet (301) auquel un piège à eau (1) est raccordé de façon détachable, dans lequel le piège à eau (1) présente un moyen de raccordement (11) pour la liaison avec le robinet (301), dans lequel le moyen de raccordement (11) se prolonge par un tuyau (10) permettant une liaison de guidage de gaz allant du système de tuyaux (300) vers le piège à eau (1), dans lequel le tuyau est relié au piège à eau (1) et dans lequel le piège à eau présente une région partielle avant (21) et une région partielle arrière (22) tournée vers l'appareil respiratoire (100), dans lequel l'appareil respiratoire présente un logement (30) pour le piège à eau (1) pour une liaison détachable avec le piège à eau (1), dans lequel le piège à eau (1) est relié à l'appareil respiratoire de manière à guider le gaz et dans lequel l'analyseur de gaz est relié au logement (30) et au piège à eau (1) de manière à guider le gaz, dans lequel l'analyseur de gaz (200) présente une unité d'aspiration (201) configurée pour transporter un flux de gaz défini hors du système de tuyaux (300) jusqu'à l'analyseur de gaz (200) en passant par le piège à eau (1), **caractérisé en ce que** le piège à eau présente une région partielle inférieure (7) formant un réservoir à eau, dans lequel la région partielle inférieure (7) présente un point de prélèvement (6) pour le prélèvement d'eau à partir du réservoir au moyen d'une canule.
